(19) Europäisches Patentamt
European Patent Office
Office européen des brevets

(11) **EP 3 838 924 A2**

(12) **EUROPEAN PATENT APPLICATION**
published in accordance with Art. 153(4) EPC

(43) Date of publication:
**23.06.2021 Bulletin 2021/25**

(51) Int Cl.:
*C07K 17/14* (2006.01)    *C07K 14/47* (2006.01)
*C12N 9/96* (2006.01)    *C12N 11/14* (2006.01)
*A61K 38/17* (2006.01)    *A61K 47/52* (2017.01)
*A61P 35/00* (2006.01)

(21) Application number: **19843288.2**

(22) Date of filing: **31.07.2019**

(86) International application number:
**PCT/KR2019/009564**

(87) International publication number:
**WO 2020/027585 (06.02.2020 Gazette 2020/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority:  **31.07.2018  US 201862712323 P**

(71) Applicant: **Lemonex Inc.**
**Seoul 08826 (KR)**

(72) Inventor: **WON, Cheol Hee**
**Seoul 08741 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **POLYPEPTIDE DELIVERY COMPOSITION**

(57)    The present invention provides a composition for delivery of polypeptide, comprising porous artificial chaperone particles having an average pore diameter of 1 to 100 nm, thereby stapling the polypeptide to immobilize a random coiled tertiary-structure of the polypeptide into a stable alpha-helix structure, and significantly improving stability and efficiency of the polypeptide.

[FIG. 17]

EP 3 838 924 A2

**Description**

[Technical Field]

The present invention relates to a composition for delivery of polypeptide.

[Background Art]

In order to maintain vital activity, various proteins in the body interact with other biopolymers such as protein-protein, protein-RNA interaction and protein-DNA interaction, and play an important role in a signaling system *in vivo.* It is often found that the interaction between specific proteins is abnormally activated, such that onset of specific diseases and their disease states are maintained. In many cases, the interaction between these polymers is through specific secondary structures of proteins such as alpha-helix and β-strand.

One of the most well-known secondary structures of proteins, the alpha-helix structure accounts for over 30% of the secondary structures found in globular proteins, and these alpha-helix structures play an essential role in interaction with biopolymers. Further, since peptides constituting the protein have a short length, it is simple to chemically synthesize the polypeptide and it is easy to manipulate the same. Further, since the polypeptide has specific activities according to structure and morphology, interest is growing in self-assembled nanostructures based on these characteristics. Therefore, short sequence peptides having the above secondary structure may be considered to have great potential as useful research tools for biological mechanism research or as drugs for treating diseases.

The peptides are stable in the protein but, when separated from the protein and present as peptides, the peptides are thermodynamically unstable, and thereby secondary structures such as helix structures are almost not maintained and have a disordered form. As a result, it is difficult to achieve high affinity and selectivity to a target. Accordingly, in order to solve the above-described problems and to be applied as a drug that can control or inhibit various interactions of lives, there is an urgent need to prepare a solution that can maintain and stabilize activated forms of the peptide or self-assembled peptide nanostructures and peptides of a folded form regardless of a changes in environment.

[Summary of Invention]

[Problems to be Solved by Invention]

An object of the present invention is to provide a composition for delivery of polypeptide that can significantly improve stability and efficiency by stapling the polypeptide.

An object of the present invention is to provide a composition for delivery of polypeptide that can significantly improve stability and efficiency by stapling the polypeptide.

[Means for Solving Problems]

To achieve the above objects, the following technical solutions are adopted in the present invention.

1. A composition for delivery of polypeptide, including: porous artificial chaperone particles having an average pore diameter of 1 to 100 nm.

2. The composition according to the above 1, wherein the particles are used to staple the polypeptide supported inside of pores thereof.

3. The composition according to the above 1, wherein an average diameter of the porous artificial chaperone particles ranges from 150 to 1000 nm.

4. The composition according to the above 1, wherein a BET surface area of the porous artificial chaperone particles ranges from 200 to 700 m$^2$/g, and a volume per gram of the pores ranges from 0.7 to 2.2 ml.

5. The composition according to the above 1, wherein the porous artificial chaperone particles are prepared by: reacting silica particles having pores with a pore diameter of less than 5 nm with a swelling agent at 120 to 180 °C for 24 to 96 hours to expand the pores having a pore diameter of less than 5 nm; and calcining the silica particles having expanded pores at a temperature of 400 °C or higher for at least 3 hours.

6. The composition according to the above 12, wherein the porous artificial chaperone particles are characterized in that t, at which an absorbance ratio in the following Equation 1 becomes 1/2, is 24 or more:

[Equation 1]

$$A_t/A_0$$

(wherein $A_0$ is absorbance of the porous artificial chaperone particles measured by putting 5 ml of suspension containing 1 mg/ml of porous artificial chaperone particles into a cylindrical permeable membrane having pores with a pore diameter of 50 kDa,

15 ml of the same solvent as the suspension comes into contact with an outside of the permeable membrane, and the inside/outside of the permeable membrane are horizontally stirred at 60 rpm and at 37 °C,

pH of the suspension is 7.4, and

$A_t$ indicates absorbance of the porous artificial chaperone particle measured after lapse of "t" hours since $A_0$ was measured) .

7. The composition according to the above 1, wherein the porous artificial chaperone particles have a hydrophilic substituent on an outer surface thereof and a hydrophobic substituent on an inside of the pores.

8. The composition according to the above 1, wherein the polypeptide is an alpha helical polypeptide or an enzyme polypeptide.

9. The composition according to the above 8, wherein the alpha helical polypeptide is at least one selected from the group consisting of Bcl-2 family protein inhibitor, MDM2/MDMX inhibitor, HIV envelope protein gp41 inhibitor, epidermal growth factor receptor inhibitor, Kirsten rat sarcoma viral oncogene homolog (KRAS) inhibitor, Rab (Ras-related in brain) inhibitor, and myoA (malaria invasion motor myosin) tail interacting protein (MTIP) inhibitor.

10. The composition according to the above 8, wherein the enzyme polypeptide is at least one selected from the group consisting of carbonic anhydrase, lysozyme, insulin, BSA, lipase, amylase, chymotrypsin, citrate synthase, formate dehydrogenase, GFP and mitochondrial malate dehydrogenase (mMDH).

11. The composition according to the above 1, wherein the polypeptide is p53.

12. A composition for delivery of polypeptide, including: porous artificial chaperone particles having an average pore diameter of 1 to 100 nm; and a polypeptide supported and stapled inside of pores of the particle.

13. The composition according to the above 12, wherein the porous artificial chaperone particles have an average diameter of 150 to 1000 nm.

14. The composition according to the above 12, wherein a BET surface area of the porous artificial chaperone particles ranges from 200 to 700 m$^2$/g and a volume per g of pores ranges from 0.7 to 2.2 ml

15. The composition according to the above 12, wherein the porous artificial chaperone particles are prepared by: reacting silica particles which have pores having a pore diameter of less than 5 nm with a swelling agent at 120 to 180 °C for 24 to 96 hours to expand the pores having a pore diameter of less than 5 nm; and calcining the silica particles having expanded pores at a temperature of 400 °C or higher for at least 3 hours.

16. The composition according to the above 12, wherein the porous artificial chaperone particles are characterized in that t, at which an absorbance ratio in the following Equation 1 becomes 1/2, is 24 or more:

[Equation 1]

$$A_t/A_0$$

(wherein $A_0$ is absorbance of the porous artificial chaperone particles measured by putting 5 ml of suspension containing 1 mg/ml of porous artificial chaperone particles into a cylindrical permeable membrane having pores with a pore diameter of 50 kDa,

15 ml of the same solvent as the suspension comes into contact with an outside of the permeable membrane, and the inside/outside of the permeable membrane are horizontally stirred at 60 rpm and at 37 °C,

pH of the suspension is 7.4, and

$A_t$ indicates absorbance of the porous artificial chaperone particle measured after lapse of "t" hours since $A_0$ was measured) .

17. The composition according to the above 12, wherein the porous artificial chaperone particles have a hydrophilic substituent on an outer surface thereof and a hydrophobic substituent on an inside of the pores.

18. The composition according to the above 12, wherein the polypeptide has an alpha-helix structure.

19. The composition according to the above 12, wherein the polypeptide is an alpha helical polypeptide or an enzyme polypeptide.

20. The composition according to the above 19, wherein the alpha helical polypeptide is at least one selected from the group consisting of Bcl-2 family protein inhibitor, MDM2/MDMX inhibitor, HIV envelope protein gp41 inhibitor, epidermal growth factor receptor inhibitor, Kirsten rat sarcoma viral oncogene homolog (KRAS) inhibitor, Rab (Ras-related in brain) inhibitor, and myoA (malaria invasion motor myosin) tail interacting protein (MTIP) inhibitor.

21. The composition according to the above 19, wherein the enzyme polypeptide is at least one selected from the group consisting of carbonic anhydrase, lysozyme, insulin, BSA, lipase, amylase, chymotrypsin, citrate synthase, formate dehydrogenase, GFP and mitochondrial malate dehydrogenase (mMDH).

22. The composition according to the above 12, wherein the polypeptide is p53.

23. A method for stapling polypeptide, including: mixing porous artificial chaperone particles which have an average pore diameter of 1 to 100 nm and polypeptide so as to carry the polypeptide inside the pores; and stapling the same.

[Advantageous Effects]

The present invention may staple a polypeptide to immobilize a random coiled tertiary-structure of the polypeptide into a stable alpha-helix structure, thereby significantly improve stability and efficiency of the polypeptide.

[Brief Description of Drawings]

FIG. 1 is micrographs of artificial chaperones based on porous nanoparticles (ACPN; hereinafter, referred to as "porous artificial chaperone particles") according to one embodiment of the present invention.

FIG. 2 is micrographs of porous artificial chaperone particles according to one embodiment of the present invention.

FIG. 3 is micrographs of small pore particles in manufacturing the porous artificial chaperone particles according to one embodiment of the present invention.

FIG. 4 is micrographs of the small pore particles according to one embodiment of the present invention.

FIG. 5 is micrographs of the porous artificial chaperone particles to pore diameter according to one embodiment of the present invention.

DDV (Degradable Delivery Vehicle) is the particles according to an embodiment, wherein the number in parenthesis means the diameter of the particle and the number of subscripts means the pore diameter. For example, DDV $(200)_{10}$ refers to a particle having a particle diameter (that is, particle size) of 200 nm and a pore diameter of 10 nm according to an embodiment.

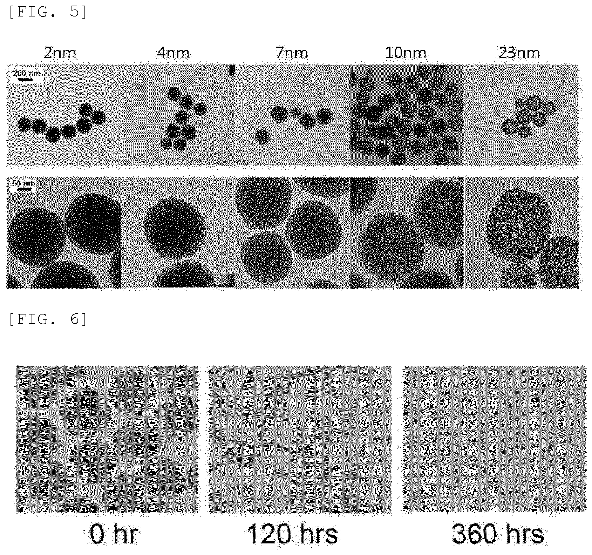

FIG. 6 is a micrograph to identify biodegradability of the porous artificial chaperone particles according to one embodiment of the present invention

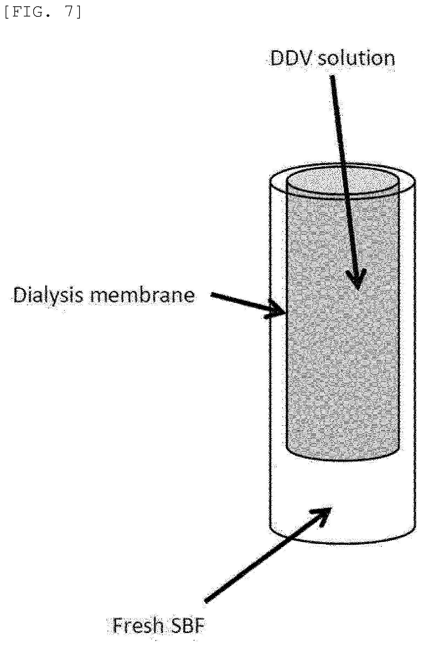

FIG. 7 a view illustrating a tube having a cylindrical permeable membrane according to one illustrative example.

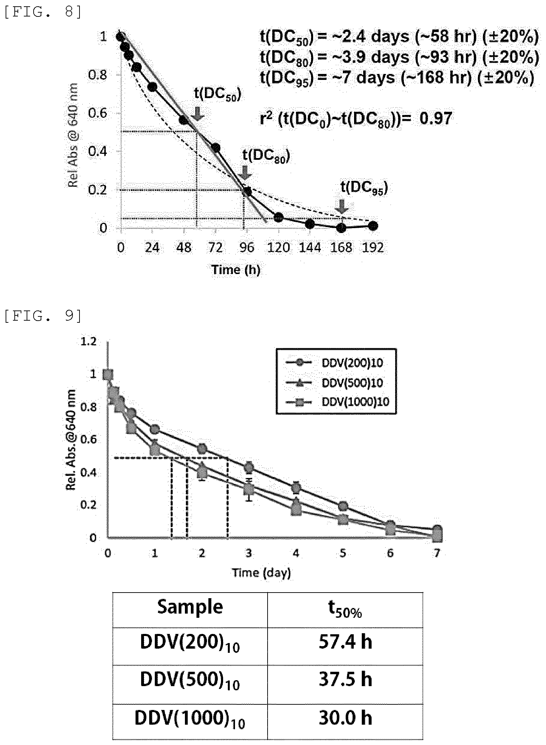

FIG. 8 is a graph illustrating results of decreasing absorbance of the porous artificial chaperone particles over time according to one embodiment of the present invention.

FIG. 9 is diagrams illustrating results of decreasing absorbance of the porous artificial chaperone particles to particle size over time according to one embodiment of the present invention

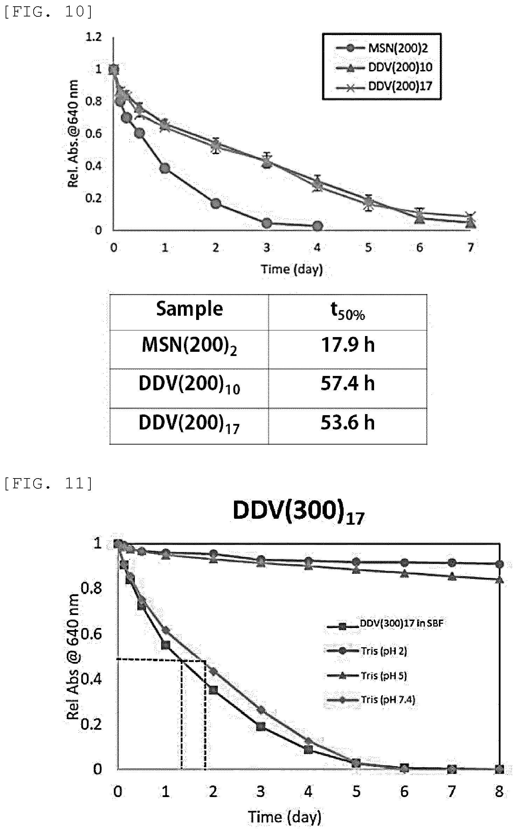

FIG. 10 is diagrams illustrating results of decreasing absorbance of the porous artificial chaperone particles to pore diameter over time according to one embodiment of the present invention.

FIG. 11 is a graph illustrating results of decreasing absorbance of the porous artificial chaperone particles for each pH of the environment over time according to one embodiment of the present invention.

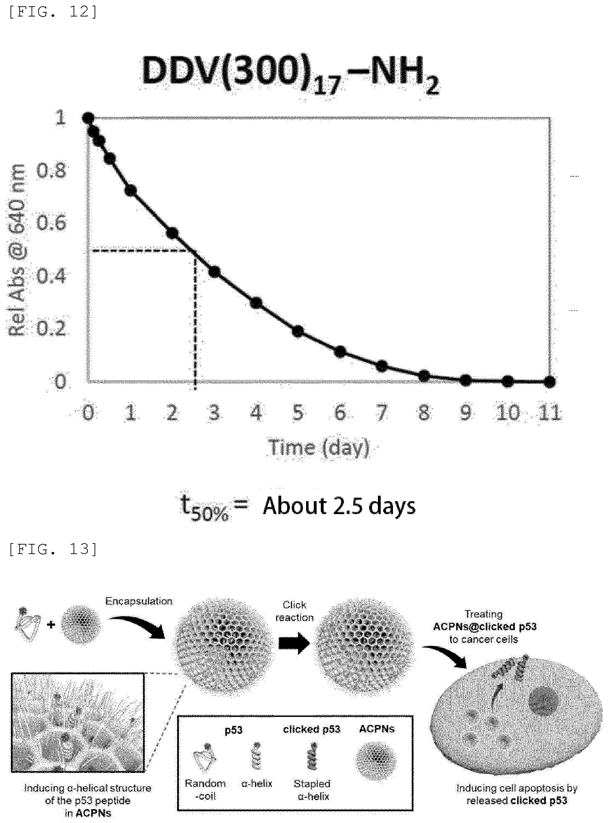

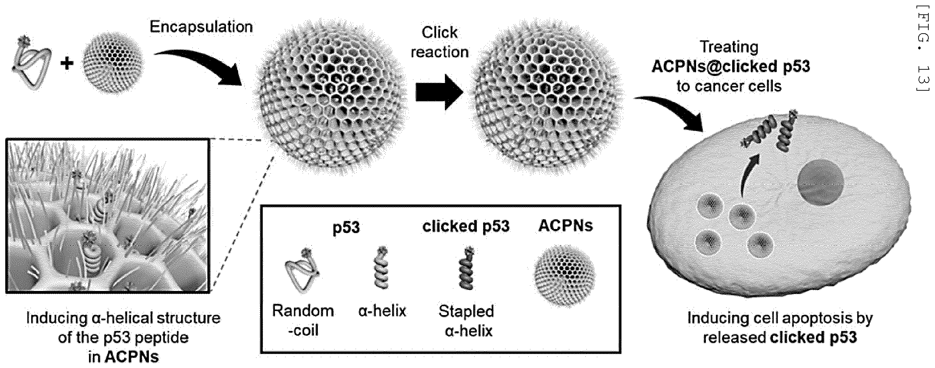

FIG. 12 is a graph illustrating results of decreasing absorbance of the porous artificial chaperone particles over time according to one embodiment of the present invention.

FIG. 13 is diagrams schematically illustrating stapling the polypeptide to porous artificial chaperone particles. As soon as the polypeptide is carried therein, click reaction is performed whereby the polypeptide is stapled to form an irreversible alpha helix structure.

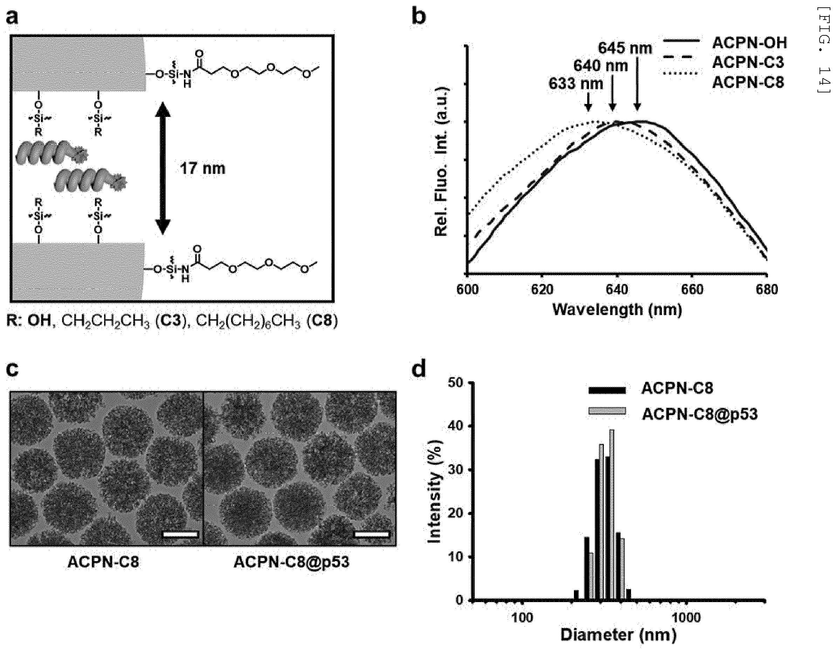

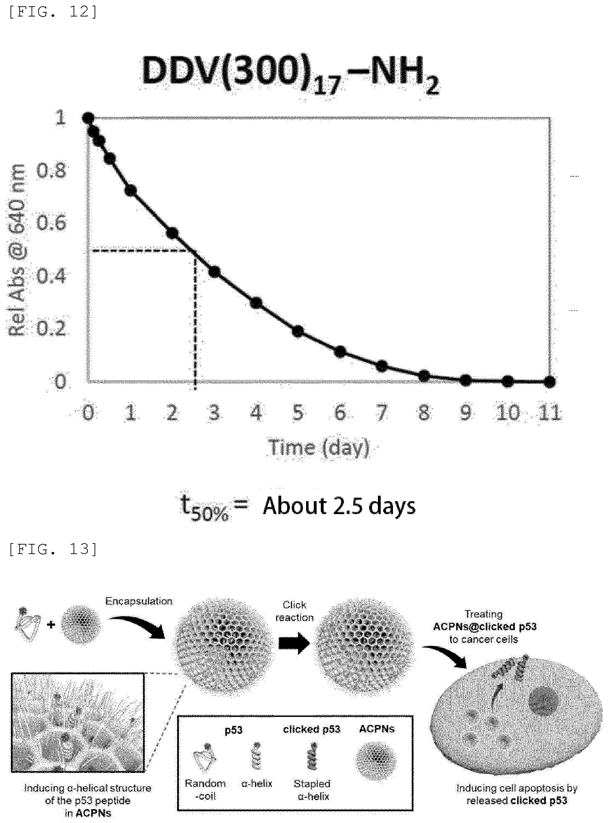

FIG. 14 is diagrams illustrating designs and characteristics of the porous artificial chaperone particles according to one embodiment of the present invention. Specifically, (a) is a design illustration of porous artificial chaperone particles. Short polyethylene glycol moieties were conjugated at an outer surface. Hydroxy, propyl and octyl groups were introduced into an inner surface. (b) is a fluorescence spectrum of Nile Red encapsulated in porous artificial chaperone particles ($\lambda$ex = 510 nm). (c) illustrates TEM images of ACPN-C8 and ACPN-C8@p53 (scale bar, 200 nm). (d) illustrates DLS data of ACPN-C8 and ACPN-C8@p53.

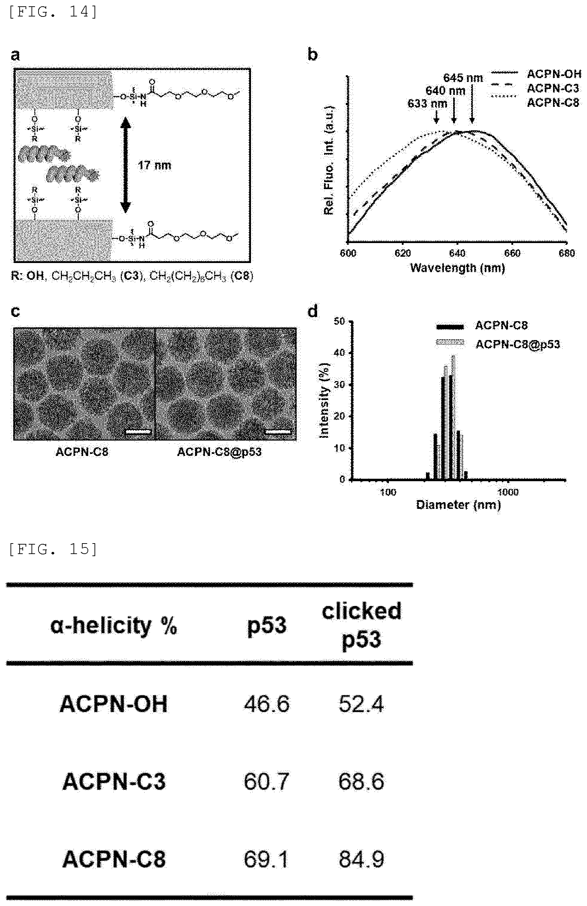

FIG. 15 is a table illustrating alpha helix values (a-helicity) when p53 is supported on the porous artificial chaperone particles according to one embodiment of the present invention. This was calculated based on the CD spectra using a CONTIN program method.

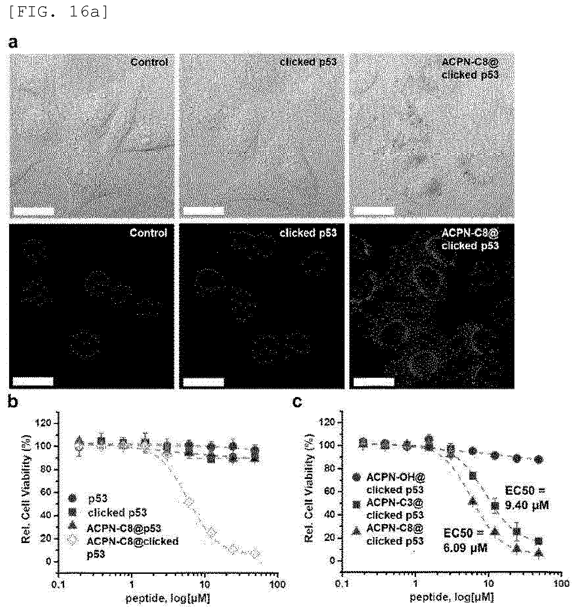

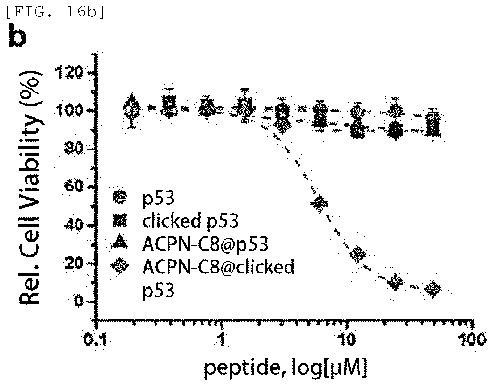

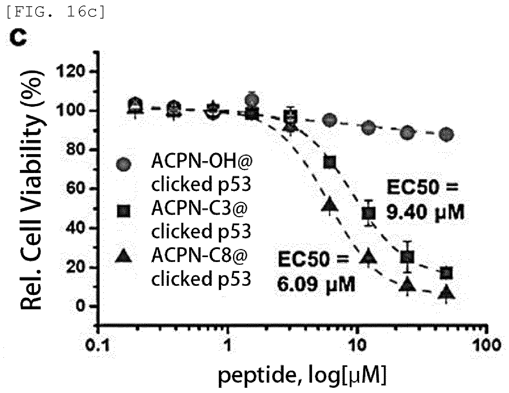

FIG. 16 is diagrams illustrating cell experiments and cell death results when p53 is supported on the porous artificial chaperone particles according to one embodiment of the present invention.

Specifically, (a) is bright field and fluorescence images showing that the clicked p53 itself and ACPN-C8@clicked p53 are absorbed into Hela Cells (bar = 25 μm). (b) illustrates cytotoxicity in HeLa cells at various concentrations of ACPN-C8@clicked p53 and comparative groups. (c) illustrates cytotoxicity in HeLa cells at various concentrations of ACPNs@clicked p53. EC50 values of ACPNs@clicked p53 were collected by a logistic curve fitting method. (d) illustrates the relative caspase-3 activity of ACPN-C8@clicked p53.

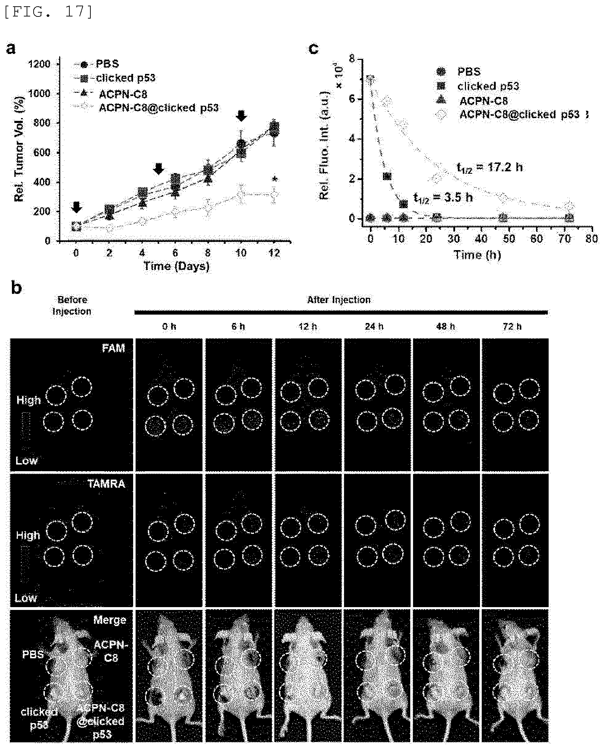

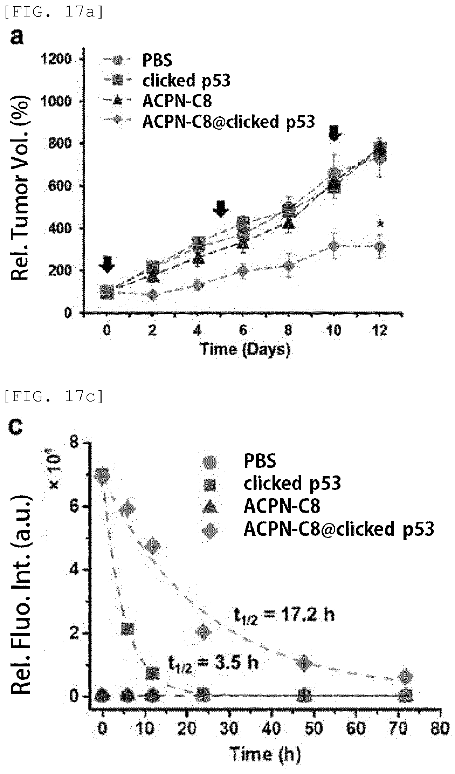

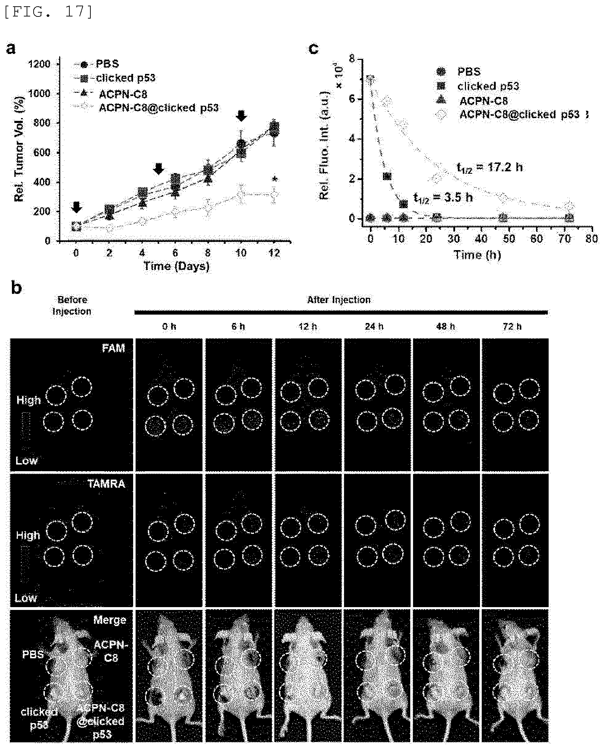

FIG. 17 is diagrams illustrating results of *in vivo* experiments when p53 is supported on the porous artificial chaperone particles according to one embodiment of the present invention. Specifically, (a) illustrates time-dependent relative tumor volumes of tumor xenograft mice that were introduced with TAMRA-labeled ACPN-C8@clicked p53 at 5 day interval and a comparative group (N = 4). (b) illustrates time-dependent whole body fluorescence images of tumor xenograft mice that were introduced with TAMRA-labeled ACPN-C8@clicked p53 and a comparative group (N = 4) (green: clicked p53, red: ACPN-C8). (c) illustrates time-dependent relative FAM fluorescence intensity of clicked p53, wherein the intensity was obtained by ROI analysis based on fluorescence images of the whole body of tumor xenograft mice.

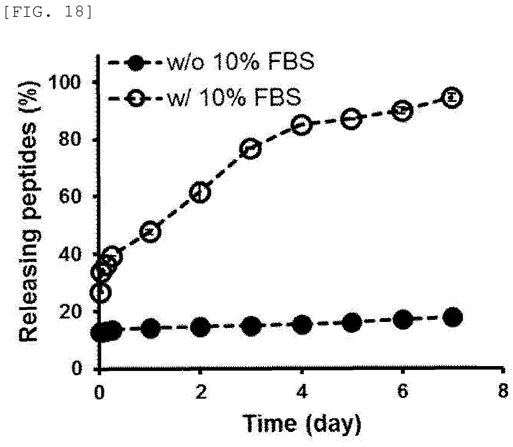

FIG. 18 is a graph illustrating results of polypeptide release evaluation of ACPN-C8@clicked p53.

[Mode for Carrying out Invention]

Hereinafter, the present invention will be described in detail.

The present invention provides a composition for delivery of polypeptide including porous artificial chaperone particles having an average pore diameter of 1 to 100 nm.

The porous artificial chaperone particles of the present invention have nano-sized pores, and may carry the polypeptide on an outer surface thereof and/or an inside of the pores.

Polypeptides (proteins) include not only chemically synthesized polypeptides but also naturally synthesized polypeptides encoded by genes of cultured cells, as well as recombinant polypeptides secreted by cells. The recombinant polypeptide may refer to peptides, polypeptides, oligopeptides and/or fusion polypeptides, and any polypeptide or a biologically active portion thereof (for example, a moiety that retains biological activity of the entire polypeptide) expressed from a recombinant genetic material encoding an amino acid. Recombinant polypeptide products may include therapeutic, prophylactic or diagnostic products.

In the present invention, stapling the polypeptide means to alter a random coil structure into an alpha helix and to maintain the structure.

When the structural disorder of the polypeptide as a drug is high, a three-dimensional structure is not fixed. Therefore, it is difficult to recognize the three-dimensional structure by a protein to be bound thereto, thereby reducing the performance of the protein as a drug.

With regard to the composition of the present invention, the nanoparticles act as chaperone particles to stabilize the polypeptide's tertiary structure, and also participate in click-stapling of the peptides in order to irreversibly maintain the alpha- helix of the polypeptides. Further, multiple pores may carry a target peptide to be delivered so as to allow the random-coiled tertiary structure to have a stable alpha-helix structure. Accordingly, the efficacy of the polypeptide may be maximized.

The polypeptide is able to be stapled in the present invention, and may be applied without limitation as long as a three-dimensional structure of the same may be unstable, and may include, for example, an alpha helical polypeptide or an enzyme polypeptide.

The alpha helical polypeptide refers to a polypeptide having an alpha-helical motif, which is formed by binding the alpha-helical motif to a hydrophobic pocket through a number of polypeptide-polypeptide interactions in the body. The alpha helical polypeptide of the present invention may have such a motif or a mimic motif. For example, the alpha helical polypeptide described above may include Bcl-2 family protein inhibitors, MDM2/MDMX inhibitor, HIV envelope protein gp41 inhibitor, epidermal growth factor receptor inhibitor, Kirsten rat sarcoma viral oncogene homolog (KRAS) inhibitor, Rab (Ras-related in brain) inhibitor, myoA (malaria invasion motor myosin) tail interacting protein (MTIP) inhibitor, and the like, more specifically p53, but it is not limited thereto.

The enzymatic polypeptide is able to be stapled into a native protein by carrying a non-native protein, and may include, for example, carbonic anhydrase, lysozyme, insulin, BSA, lipase, amylase, chymotrypsin, citrate synthase, formate dehydrogenase, GFP, mitochondrial malate dehydrogenase (mMDH), and the like, but it is not limited thereto.

The composition of the present invention may be used to deliver the supported polypeptide into an individual, that is, a subject. In this regard, the polypeptide used herein may include any one as long as it has the same species-derived sequence as the subject to be delivered or is applicable to the corresponding subject even though the polypeptide is derived from other species.

Porous artificial chaperone particles of the present invention are biodegradable particles. In such a case, the chaperone particles may be biodegraded to release a polypeptide when the chaperone particles carry the polypeptide and are administered into the body. The porous artificial chaperone particles of the present invention may be slowly degraded in the body to allow the supported polypeptide to be released in a sustained manner. For example, t, at which an absorbance ratio in the following Equation 1 becomes 1/2, is 24 or more:

$$[\text{Equation 1}]$$

$$A_t / A_0$$

(wherein $A_0$ is absorbance of the porous artificial chaperone particles measured by putting 5 ml of suspension containing 1 mg/ml of porous artificial chaperone particles into a cylindrical permeable membrane having pores with a pore diameter of 50 kDa,
15 ml of the same solvent as the suspension comes into contact with an outside of the permeable membrane, and the inside/outside of the permeable membrane are horizontally stirred at 60 rpm and at 37 °C,
pH of the suspension is 7.4, and
$A_t$ indicates absorbance of the porous artificial chaperone particle measured after lapse of "t" hours since $A_0$ was measured).

The above Equation 1 means what a rate the porous artificial chaperone particles are degraded in an environment similar to the body.

As shown in FIG. 7, for example, absorbances $A_0$ and $A_t$ in the above Equation 1 may be measured after placing porous artificial chaperone particles and a suspension in a cylindrical permeable membrane and also placing the same suspension outside the permeable membrane.

The porous artificial chaperone particles of the present invention are biodegradable, and may be slowly degraded in the suspension. The diameter of 50 kDa corresponds to about 5 nm, which allows biodegradable porous artificial chaperone particles to pass through a permeable membrane having a diameter of 50 kDa, and a cylindrical permeable membrane is under horizontal agitation at 60 rpm to evenly blend the suspension, such that the degraded porous artificial chaperone particles can come out of the permeable membrane.

The absorbance in the above Equation 1 may be measured, for example, under an environment in which the suspension outside the permeable membrane is replaced with a new suspension. The suspension may be continuously replaced, or replaced every period wherein the period is periodic or irregular. For example, the suspension may be replaced at 1 hour interval, 2 hours interval, 3 hours interval, 6 hours interval, 12 hours interval, 24 hours interval, 2 days interval, 3 days interval, 4 days interval, 7 days interval, etc., within a range of 1 hour to 1 week, but it is not limited thereto.

The absorbance ratio of 1/2 means that the absorbance is half of the initial absorbance after t hours, that is, that approximately half of the porous artificial chaperone particles are degraded.

The suspension may be a buffer solution, for example, at least one selected from the group consisting of phosphate buffered saline (PBS) and simulated body fluid (SBF), and more specifically, PBS.

"t" in the above Equation 1 of the present invention, at which the absorbance ratio becomes 1/2, may be 24 or more, for example, t may range from 24 to 120. That is, within the above range, t may range from 24 to 96, 24 to 72, 30 to 70, 40 to 70, 50 to 65, etc., but it is not limited thereto.

With regard to the porous artificial chaperone particles of the present invention, t at which the absorbance ratio in the above Equation 1 becomes 1/5 may range from 70 to 140. For example, t may range from 80 to 140, 80 to 120, 80 to 110, 70 to 140, 70 to 120, 70 to 110, etc. within the above range, but it is not limited thereto.

With regard to the porous artificial chaperone particles of the present invention, t at which the absorbance ratio in the above Equation 1 becomes 1/20 may range from 130 to 220. For example, t may range from 130 to 200, 140 to 200, 140 to 180, 150 to 180, etc. within the above range, but it is not limited thereto.

With regard to the porous artificial chaperone particles of the present invention, t at which the absorbance ratio in the above Equation 1 becomes 0.01 or less may be 250 or more. For example, t may be 300 or more, 350 or more, 400 or more, 500 or more, 1000 or more, etc. and the upper limit may be 2000, but it is not limited thereto.

With regard to the porous artificial chaperone particles of the present invention, the absorbance ratio and t in the above Equation 1 have high positive correlation. For example, Pearson correlation coefficient may be 0.8 or more, and for example, 0.9 or more and 0.95 or more.

"t" in the above Equation 1 means how fast the porous artificial chaperone particles are degraded under the environment similar to the body. That is, t may be regulated by adjusting, for example, a surface area, a particle size, a pore diameter, substituents on the surface of the porous artificial chaperone particles and/or the inside of the pores, compactness of the surface and the like.

For example, the surface area of the particles may be increased to reduce t, or the surface area may be decreased to increase t. The surface area may be regulated by adjusting the particle size and the pore diameter of the particles. Further, if direct exposure of the porous artificial chaperone particles to the environment (such as solvents) is reduced by placing substituents on the surface of the particles and/or the inside of the pores, t may be increased. Further, when the porous artificial chaperone particles support or carry the polypeptide, and when increasing affinity between the polypeptide and the porous artificial chaperone particles, direct exposure of the porous artificial chaperone particles to the environment may be reduced, thereby increasing t. In addition, t may be increased by preparing the particles with more compact surface. As described above, various examples of adjusting t in the above Equation 1 have been described, but it is not limited thereto.

The porous artificial chaperone particles of the present invention may have a spherical shape, but it is not limited thereto.

The porous artificial chaperone particles of the present invention may have an average diameter of, for example, 150 to 1000 nm. For example, the average diameter may range from 150 to 800 nm, 150 to 500 nm, 150 to 400 nm, 150 to 300 nm, and 150 to 200 nm, etc. within the above range, but it is not limited thereto.

The porous artificial chaperone particles of the present invention may have an average pore diameter of, for example, 1 to 100 nm. For example, the average diameter may range from 5 to 100 nm, 7 to 100 nm, 7 to 50 nm, 10 to 50 nm, 10 to 30 nm, 7 to 30 nm, etc., within the above range, but it is not limited thereto. The chaperone particles having a larger diameter as described above may carry a large amount of polypeptide, and may further carry large-sized polypeptides.

The porous artificial chaperone particles of the present invention may have a BET surface area of, for example, 200 to 700 $m^2/g$. For example, the BET surface area may range from 200 to 700 $m^2/g$, 200 to 650 $m^2/g$, 250 to 650 $m^2/g$, 300 to 700 $m^2/g$, 300 to 650 $m^2/g$, 300 to 600 $m^2/g$, 300 to 550 $m^2/g$, 300 to 500 $m^2/g$, 300 to 450 $m^2/g$, etc. within the above range, but it is not limited thereto.

Porous silica nanoparticles of the present invention may have a volume per gram, for example, 0.7 to 2.2 ml. For example, the volume may range from 0.7 to 2.0 ml, 0.8 to 2.2 ml, 0.8 to 2.0 ml, 0.9 to 2.0 ml, 1.0 to 2.0 ml, etc. within the above range, but it is not limited thereto. If the volume per gram is too small, a degradation rate may be too high. Further, it is difficult to manufacture excessively large particles or particles having an intact shape.

The porous artificial chaperone particles of the present invention may have hydrophilic substituents and/or hydrophobic substituents on an outer surface thereof and/or an inside of the pores. For example, only hydrophilic substituents or only hydrophobic substituents may exist on both the surface of the particles and inside of the pores, hydrophilic substituents or hydrophobic substituents may be present on either the surface of the particles or the inside of the pores, or hydrophilic substituents may be present on the surface of the particles while hydrophobic substituents may exist inside of the pores, or vice versa.

Release of the polypeptide supported on the porous artificial chaperone particles according to the present invention is mainly performed by degradation of nanoparticles. Specifically, interaction of the porous artificial chaperone particles with the release environment of the polypeptide is adjusted to regulate a degradation rate of the nanoparticles, so that a release rate of the polypeptide can be regulated. Further, the polypeptide may be diffused and released from the nanoparticles, wherein adjusting substituents may regulate a binding force of the polypeptide to the nanoparticles, thereby controlling release of the polypeptide.

Further, in order to increase a binding force of the porous artificial chaperone particle to a poorly soluble (hydrophobic) polypeptide, hydrophobic substituents may be present inside of the pores of the particle. Further, in aspects of easy use and formulation, the surface of the particles may also be treated to have hydrophilic substituents.

Specifically, the porous artificial chaperone particles may have a hydrophilic substituent on the surface of the particles and a hydrophobic substituent on the inside of the pores. Such porous artificial chaperone particles are preferable in view of excellent dispersibility and carried polypeptide.

The hydrophilic substituents may include, for example, hydroxyl group, carboxy group, amino group, carbonyl group, sulfhydryl group, phosphate group, thiol group, ammonium group, ester group, imide group, thioimide group, keto group, ether group, indene group, sulfonyl group, polyethyleneglycol group and the like. Further, the hydrophobic substituent may include, for example, substituted or unsubstituted C1 to C30 alkyl group, substituted or unsubstituted C3 to C30 cycloalkyl group, substituted or unsubstituted C6 to C30 aryl group, substituted or unsubstituted C2 to C30 heteroaryl group, halogen group, C1 to C30 ester group, halogen-containing group and the like.

Further, the porous artificial chaperone particles of the present invention may be positively charged and/or negatively charged at the outer surface of the particles and/or the inside of the pores. For example, both the surface of the particles and the inside of the pores may be positively charged or negatively charged, and only the surface of the particles or the inside of the pores may be positively charged or negatively charged. Alternatively, the surface of the particle may be positively charged while the inside of the pore may be negatively charged or vice versa, or vice versa.

The charging may be performed, for example, by the presence of a cationic substituent or an anionic substituent.

The cationic substance may include, for example, an amino group or any other nitrogen-containing group. Further, the anionic substituent may include, for example, a carboxy group (-COOH), sulfonic acid group (-SO$_3$H), thiol group (-SH), etc. as an acidic group, but it is not limited thereto.

Likewise, when interaction of the porous artificial chaperone particles with release environment of a polypeptide is regulated by adjusting the substituents through charging, a degradation rate of nanoparticles may be regulated to control a release rate of the polypeptide. Further, the polypeptide may be diffused and released from the nanoparticles. In this regard, adjusting the substituents may regulate a binding force of the polypeptide to the nanoparticles, thereby controlling release of the polypeptide.

Further, the porous artificial chaperone particles of the present invention may include substituents for the purposes of: supporting the polypeptide on the surface of the particles and/or inside of the pores; delivery of the polypeptide into a target cell; supporting other substances for other purposes; or binding of additional substituents. Further, the porous artificial chaperone particles may also include antibodies, ligands, cell permeable peptides, or aptamers bound thereto.

The substituents on the surface of the particles and/or the inside of the pores, charge, binders, etc. described above may be added by, for example, surface modification.

Surface modification may be performed, for example, by reacting a compound having a substituent to be introduced with the particles, wherein the compound may be, for example, alkoxysilane having C1 to C10 alkoxy group, but it is not limited thereto. The alkoxysilane has one or more alkoxy groups, for example, 1 to 3 alkoxy groups. Further, there may be a substituent to be introduced into a site where the alkoxy group is not bound, or a substituent substituted with the same.

The porous artificial chaperone particles of the present invention may be manufactured, for example, through small pore particle preparation and pore expansion processes and, if necessary, may be manufactured further through calcination, or surface modification process and the like. If both the calcination and the surface modification processes have been implemented, the particles may be surface-modified after calcination.

The small pore particles may be, for example, particles having an average pore diameter of 1 to 5 nm.

The small pore particles may be harvested by adding a surfactant and a silica precursor in a solvent, followed by agitation and homogenization.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, $\gamma$-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

When using a mixed solvent of water and the organic solvent, a relative ratio of water and organic solvent may be, for example, in a volume ratio of 1:0.7 to 1.5, for example, 1:0.8 to 1.3, but it is not limited thereto.

The surfactant may include, for example, cetyltrimethylammonium bromide (CTAB), hexadecyltrimethylammonium bromide (TMABr), hexadecyltrimethylpyridinium chloride (TMPrCl), tetramethylammonium chloride (TMACl), etc., and specifically, CTAB may be used.

The surfactant may be added, for example, in an amount of 1 to 10 g, for example, 1 to 8 g, 2 to 8 g or 3 to 8 g per liter of solvent, but it is not limited thereto.

The silica precursor may be added after stirring with addition of a surfactant to the solvent. The silica precursor may be, for example, tetramethyl orthosilicate (TMOS), but it is not limited thereto.

The stirring may be conducted, for example, for 10 to 30 minutes, but it is not limited thereto

The silica precursor may be added in an amount of 0.5 to 5 ml per liter of solvent, for example, 0.5 ml to 4 ml, 0.5 to 3 ml, 0.5 to 2 ml, 1 to 2 ml, etc. within the above range, but it is not limited thereto.

If necessary, sodium hydroxide may further be used as a catalyst, specifically, and may be added under stirring after addition of the surfactant and before addition of the silica precursor to the solvent.

The sodium hydroxide may be added in an amount of 0.5 to 8 ml per liter of solvent, for example, 0.5 to 5 ml, 0.5 to 4 ml, 1 to 4 ml, 1 to 3 ml, 2 to 3 ml, etc. within the above range with respect to 1 M aqueous sodium hydroxide solution, but it is not thereto.

After addition of the silica precursor, the solution may be reacted with stirring. The stirring may be conducted for 2 to 15 hours, for example, 3 to 15 hours, 4 to 15 hours, 4 to 13 hours, 5 to 12 hours, 6 to 12 hours, 6 to 10 hours, etc. within the above range, but it is not limited thereto. If the stirring time (reaction time) is too short, nucleation may be insufficient.

After agitation, the solution may be aged. Aging may be performed for 8 to 24 hours, for example, for 8 to 20 hours, 8 to 18 hours, 8 to 16 hours, 8 to 14 hours, 10 to 16 hours, 10 to 14 hours, etc. within the above range, but it is not limited thereto.

Thereafter, the reaction product may be washed and dried to harvest porous artificial chaperone particles and, if necessary, separation of unreacted material may proceed before washing.

Separation of the unreacted material may be implemented by separating the supernatant, for example, through centrifugation. For example, centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous artificial chaperone particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

Thereafter, the pore of the harvested porous artificial chaperone particles may be expanded, and such pore expansion may be conducted using a pore swelling agent.

The pore swelling agent may include, for example, trimethylbenzene, triethylbenzene, tripropylbenzene, tributylbenzene, tripentylbenzene, trihexylbenzene, toluene, benzene, etc., and specifically, trimethylbenzene may be used, but it is not limited thereto.

Further, the pore swelling agent used herein may be, for example, N, N-dimethylhexadecylamine (DMHA), but it is not limited thereto.

The pore expansion may be performed, for example, by mixing the porous artificial chaperone particles in the solvent with a pore swelling agent and heating the mixture to induce reaction.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The porous artificial chaperone particles may be added in a ratio of 10 to 200 g per liter of solvent, for example, 10 to 150 g, 10 to 100 g, 30 to 100 g, 40 to 100 g, 50 to 100 g, 50 to 80 g, 60 to 80 g, etc., within the above range, but it is not limited thereto.

The porous artificial chaperone particles may be evenly dispersed in a solvent. For example, the porous artificial chaperone particles may be added to the solvent and ultrasonically dispersed. In the case of using a mixed solvent, the porous artificial chaperone particles may be dispersed in a first solvent, followed by adding a second solvent thereto.

The pore swelling agent may be added in a ratio of 10 to 200 parts by volume ("vol. parts") to 100 vol. parts of solvent, for example, 10 to 150 vol. parts, 10 to 100 vol. parts, 10 to 80 vol. parts, 30 to 80 vol. parts, 30 to 70 vol. parts, etc. within the above range, but it is not limited thereto.

The reaction may be carried out at 120 to 190 °C, for example, 120 to 190 °C, 120 to 180 °C, 120 to 170 °C, 130 to 170 °C, 130 to 160 °C, 130 to 150 °C, 130 to 140 °C, etc. within the above range, but it is not limited thereto.

The reaction may be carried out for 6 to 96 hours, for example, 30 to 96 hours, 30 to 96 hours, 30 to 80 hours, 30 to 72 hours, 24 to 80 hours, 24 to 72 hours, 36 to 96 hours, 36 to 80 hours, 36 to 72 hours, 36 to 66 hours, 36 to 60 hours, 48 to 96 hours, 48 to 88 hours, 48 to 80 hours, 48 to 72 hours, 6 to 96 hours, 7 to 96 hours, 8 to 80 hours, 9 to 72 hours, 9 to 80 hours, 6 to 72 hours, 9 to 96 hours, 10 to 80 hours, 10 to 72 hours, 12 to 66 hours, 13 to 60 hours, 14 to 96 hours, 15 to 88 hours, 16 to 80 hours, 17 to 72 hours, etc. within the above range, but it is not limited thereto.

The time and temperature may be desirably adjusted within the ranges exemplified above so that the reaction may be carried out sufficiently but not excessively. For example, when the reaction temperature is reduced, the reaction time may be increased, and when the reaction temperature is increased, the reaction time may be shortened. If the reaction is not sufficiently performed, pore expansion may be insufficient. On the other hand, if the reaction proceeds excessively, the particles may collapse due to overexpansion of the pores.

The reaction may be carried out, for example, by gradually raising the temperature. Specifically, the reaction may be carried out by gradually raising the temperature at a rate of 0.5 to 15 °C/min from the room temperature to the above-defined temperature. For example, the temperature may be raised at a rate of 1 to 15 °C/min, 3 to 15 °C/min, 3 to 12 °C/min, 3 to 10 °C/min, etc., but it is not limited thereto.

The reaction may be carried out under stirring. For example, the stirring may be implemented at a speed of 100 rpm or more, and specifically, at a speed of 100 to 1000 rpm, but it is not limited thereto.

After the reaction, the reaction solution may be cooled slowly, for example, by gradually decreasing the temperature. Specifically, the reaction may be carried out by gradually decreasing the temperature at a rate of 0.5 to 20 °C/min from the above-defined temperature to room temperature. For example, the temperature may be decreased at a rate of 1 to 20 °C/min, 3 to 20 °C/min, 3 to 12 °C/min, 3 to 10 °C/min, etc. within the above range, but it is not limited thereto.

After cooling, the reaction product may be washed and dried to harvest porous artificial chaperone particles having expanded pores. If necessary, unreacted material may be first separated before washing.

Separation of the unreacted material may be implemented by separating the supernatant, for example, through centrifugation. Herein, centrifugation may be conducted, for example, at 6,000 to 10,000 rpm, and the centrifugation time may range from 3 minutes to 60 minutes. For example, the centrifugation may be conducted for 3 to 30 minutes, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times, 4 times, 5 times, 6 times, 7 times, 8 times, etc.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous artificial chaperone particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

Thereafter, the harvested particles may be subjected to calcination, which is a process of heating the particles to remove silanol groups present on the surface of the particles and inside of the pores so as to reduce reactivity of the particles, provide a more compact structure, and remove organic matter filling the pores. For example, the particles may be heated to a temperature of 400 °C or higher. The upper limit of the temperature is not particularly limited but may be 1000 °C, 900 °C, 800 °C, 700 °C, etc. The heating may be conducted, for example, for 3 hours or more. The upper limit of the heating time is not particularly limited but may be 24 hours, 12 hours, 10 hours, 8 hours, 6 hours, etc. More particularly, the heating may be conducted at 400 to 700 °C for 3 to 8 hours or at 500 to 600 °C for 4 to 5 hours, but it is not limited thereto.

Removing the organic matter filling the pores can prevent some problems of cytotoxicity or foaming caused by the remaining organic matter.

Then, the harvested porous artificial chaperone particles may be subjected to surface modification, and the surface modification may be performed on the surface of the particles and/or the inside of the pores. Both the particle surface and the inside of the pores may be surface-modified in the same manner, or may be surface-modified differently.

The particles may be charged or have hydrophilic and/or hydrophobic properties through surface modification.

More specifically, in order to effectively support the polypeptides, surface modification of the porous artificial chaperone particles may be performed by having at least one substituent selected from the group consisting of amino, aminoalkyl, alkylamino, heterocyclic aromatic compound group containing a nitrogen atom, cyan and guanidine groups.

Surface modification may be performed, for example, by reacting a compound having a hydrophilic, hydrophobic, cationic or anionic substituent to be introduced with the particles, wherein the compound may be, for example, alkoxysilane having a C1 to C10 alkoxy group, but it is not limited thereto.

The alkoxysilane has one or more alkoxy groups, for example, 1 to 3 alkoxy groups. Further, there may be a substituent to be introduced into a site where the alkoxy group is not bound, or a substituent substituted with the same.

When alkoxysilane reacts with the porous artificial chaperone particles, a covalent bond is formed between a silicon atom and an oxygen atom so that the alkoxysilane may be bound to the surface of the silicone particles and/or the inside of the pores. Since the alkoxysilane has a substituent to be introduced, the corresponding substituent may be introduced into the surface of the porous artificial chaperone particles and/or the inside of the pores.

The reaction may be carried out by reacting the porous artificial chaperone particles dispersed in a solvent with alkoxysilane.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, $\gamma$-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The positively charging may be performed by reacting the porous artificial chaperone particles with alkoxysilane having a basic group such as a nitrogen-containing group, for example, an amino group or an aminoalkyl group. Specifically, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silane, 3-(2-aminoethylamino)propyldimethoxymethylsilane, etc. may be used, but it is not limited thereto.

The negatively charging may be performed by reacting the porous artificial chaperone particles with alkoxysilane having an acidic group such as a carboxyl group, a sulfonic acid group, a thiol group, etc. Specifically, (3-Mercaptopropyl)trimethoxysilane may be used, but it is not limited thereto.

The hydrophilic property may be obtained by reacting the porous artificial chaperone particles with alkoxysilane having a hydrophilic group, for example, hydroxyl group, carboxy group, amino group, carbonyl group, sulfhydryl group, phosphate group, thiol group, ammonium group, ester group, imide group, thioimide group, keto group, ether group, indene group, sulfonyl group, polyethyleneglycol group and the like. Specifically, N-[3-(trimethoxysilyl)propyl]ethylenediamine, N1-(3-trimethoxysilylpropyl)diethylenetriamine, (3-aminopropyl)trimethoxysilane, (3-mercaptopropyl) trimethoxysilane, trimethoxy[3-(methylamino)propyl]silane, 3-(2-aminoethylamino)propyldimethoxymethylsilane may be used, but it is not limited thereto.

The hydrophobic property may be obtained by reacting the porous artificial chaperone particles with alkoxysilane having a hydrophobic substituent, for example, substituted or unsubstituted C1 to C30 alkyl group, substituted or unsubstituted C3 to C30 cycloalkyl group, substituted or unsubstituted C6 to C30 aryl group, substituted or unsubstituted C2 to C30 heteroaryl group, halogen group, C1 to C30 ester group, halogen-containing group and the like. Specifically, trimethoxy(octadecyl)silane, trimethoxy-n-octylsilane, trimethoxy(propyl)silane, isobutyl(trimethoxy)silane, trimethoxy(7-octen-1-yl)silane, trimethoxy(3,3,3-trifluoropropyl)silane, trimethoxy(2-phenylethyl)silane, vinyltrimethoxysilane, cyanomethyl, 3-(trimethoxysilyl)propyl]trithiocarbonate, (3-bromopropyl)trimethoxysilane, etc. may be used, but it is not limited thereto.

Further, in order to increase a binding force of the porous artificial chaperone particle to a poorly soluble (hydrophobic) polypeptide, hydrophobic substituents may be present inside of the pores of the particle. Further, in aspects of easy use and formulation, the surface of the particle may also be treated to have hydrophilic substituents.

Further, the surface modification may be performed in combination. For example, surface modification may be performed twice or more on the outer surface of the particles or the inside of the pores. As a specific example, a compound including a carboxyl group may be bound to silica particles having amino groups introduced therein through amide bond in order to change the positively-charged particles to have different surface properties, but it is not limited thereto.

The reaction of the porous artificial chaperone particles with alkoxysilane may be carried out, for example, under heating. The heating may be conducted at 80 to 180 °C, for example, 80 to 160 °C, 80 to 150 °C, 100 to 160 °C, 100 to 150 °C, 110 to 150 °C, etc. within the above range, but it is not limited thereto.

The reaction of the porous artificial chaperone particles with alkoxysilane may be carried out for 4 to 20 hours, for example, 4 to 18 hours, 4 to 16 hours, 6 to 18 hours, 6 to 16 hours, 8 to 18 hours, 8 to 16 hours, 8 to 14 hours, 10 to 14 hours, etc. within the above range, but it is not limited thereto.

The reaction temperature, time and an amount of the compound used for surface modification may be desirably selected according to an extent of surface modification. In other words, reaction conditions will vary depending on hydrophilic property, hydrophobic property and a level of charge with regard to a polypeptide. Specifically, by controlling hydrophilic property, hydrophobic property and/or the level of charge of the porous artificial chaperone particles, a release rate of the polypeptide may be controlled. For example, if the polypeptide has strong negative charge at neutral pH, the reaction temperature may be raised, the reaction time may be extended or the amount of the treated compound may be increased so as to make the porous artificial chaperone particles to have strong positive charge, but it is not limited thereto.

Further, the porous artificial chaperone particles of the present invention may be manufactured through, for example, preparation of small pore particles, pore expansion, surface modification, and internal pore modification.

Preparation of small pore particles and pore expansion may be performed by the above-described processes and, after preparation of the small pore particles and after pore expansion, washing and drying processes may be implemented.

If necessary, unreacted materials may be separated before washing, and separation of the unreacted materials may be conducted by separating the supernatant through centrifugation.

Centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing after preparation of small pore particles may be conducted by a method/condition within the above-described range, but it is not limited thereto.

The washing after pore expansion may be conducted under more moderate conditions than the above embodiments. For example, washing may be conducted three times or less, but it is not limited thereto.

The surface modification and internal pore modification may be performed by the above-described processes, respectively. Herein, surface modification and then internal pore modification may be performed in this order, and a washing process may be further conducted between the above two processes.

When the washing is conducted in more moderated conditions after preparation of small pore particles and pore expansion, a reaction solution such as a surfactant used for particle production and pore expansion is filled in the pores so that the inside of the pores is not modified during surface modification and, instead, only the surface of the particles may be modified. Thereafter, the reaction solution inside of the pores may be washed out and removed.

Particle washing between the surface modification and the internal pore modification processes may be carried out using water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The washing may be carried out under centrifugation, for example at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous artificial chaperone particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

The polypeptide may be supported on the surface of the porous artificial chaperone particles and/or inside of the pores. Herein, the supporting may be performed, for example, by mixing porous artificial chaperone particles in a solvent with the polypeptide.

The solvent may be water and/or an organic solvent, and the solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

Further, PBS (phosphate buffered saline solution), SBF (simulated body fluid), borate-buffered saline, tris-buffered saline may be used as the solvent.

The polypeptide supported on the porous artificial chaperone particles may be gradually released over an extended time. Such sustained release may be continuous or discontinuous, linear or nonlinear. Further, the release may vary depending on characteristics of the porous artificial chaperone particles and/or interaction between the porous artificial chaperone particles and the polypeptide.

The polypeptide supported on the porous artificial chaperone particles is released when the porous artificial chaperone particles are biodegraded. Specifically, the porous artificial chaperone particles according to the present invention are slowly degraded to allow release of the supported polypeptide in a sustained manner. Such release may be controlled by, for example, adjusting surface area, particle size, pore diameter, substituent on the surface of the particles and/or inside of the pores, surface compactness, etc. with regard to the porous artificial chaperone particles, but it is not limited thereto.

The polypeptide supported on the porous artificial chaperone particles may be released while being separated and diffused from the porous artificial chaperone particles. Since release is influenced by correlation between the porous artificial chaperone particles, the polypeptide, and release environment of the same. Therefore, regulating the correlations may control the release of the polypeptide. For example, by enhancing or weakening a binding force of the porous artificial chaperone particles to the polypeptide through surface modification, the release of the polypeptide may be controlled.

More specifically, in the case where the supported polypeptide is poorly soluble (hydrophobic), the surface of the particles and/or the inside of the pores have hydrophobic substituents so as to increase a binding force of the porous artificial chaperone particles to the polypeptide, whereby the polypeptide may be released in a sustained manner. For example, the porous artificial chaperone particles may be surface-modified with alkoxysilane having a hydrophobic substituent.

As used herein, the term "poorly soluble" means to be insoluble, practically insoluble or only slightly soluble (in water), which is a word defined in "Pharmaceutical Science" 18th Edition (issued by U.S.P., Remington, Mack Publishing Company).

The poorly soluble material may have, for example, water solubility of less than 10 g/L, specifically, less than 5 g/L, and more specifically, less than 1 g/L at 1 atmosphere and 25 °C, but it is not limited thereto.

When the supported polypeptide is water-soluble (hydrophilic), the surface of the particles and/or the inside of the pores have hydrophilic substituents so as to increase a binding force of the porous artificial chaperone particles to the polypeptide, whereby the polypeptide may be released in a sustained manner. For example, the porous artificial chaperone particles may be surface-modified with alkoxysilane having a hydrophilic substituent.

For example, the water-soluble material may have a water solubility of 10 g/L or more at 1 atmosphere and 25 °C, but it is not limited thereto.

In the case where the supported polypeptide is charged, the surface of the particles and/or the inside of the pores are charged with opposite charges, so as to increase a binding force of the porous artificial chaperone particles to the polypeptide, whereby the polypeptide may be released in a sustained manner. For example, the porous artificial chaperone particles may be surface-modified with alkoxysilane having an acidic group or a basic group.

Specifically, if the polypeptide is positively charged at neutral pH, the surface of the particles and/or the inside of the pores may be negatively charged at neutral pH so as to increase a binding force of the porous artificial chaperone particles to the polypeptide, whereby the polypeptide may be released in a sustained manner. For example, the porous artificial chaperone particles may be surface-modified with alkoxysilane having an acidic group such as a carboxyl group (-COOH), a sulfonic acid group ($-SO_3H$) , etc.

Further, if the polypeptide is negatively charged at neutral pH, the surface of the particles and/or the inside of the pores may be positively charged at neutral pH, so as to increase a binding force of the porous artificial chaperone particles to the polypeptide, whereby the polypeptide may be released in a sustained manner. For example, the porous artificial chaperone particles may be surface-modified with alkoxysilane having a basic group such as an amino group or any other nitrogen-containing group.

The polypeptide may be released for a period of, for example, 7 days to 1 year or more, depending on types of treatment to be required, release environments and types of porous artificial chaperone particles to be used.

Further, if the porous artificial chaperone particles of the present invention are biodegradable, these can be 100% degraded. Therefore, the polypeptide supported thereon may be 100% released.

The pharmaceutical composition of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be added to formulate the pharmaceutical composition into injectable formulations, pills, capsules, granules or tablets such as aqueous solutions, suspensions, emulsions and the like.

The pharmaceutical composition of the present invention is applicable in a form of any formulation and may be prepared in oral or parenteral formulations. The pharmaceutical formulations of the present invention may include forms suitable for oral, rectal, nasal, topical (including the cheek and sublingual), subcutaneous, vaginal or parenteral (intramuscular, subcutaneous) administration. Alternatively, forms suitable for administration by inhalation or insufflations may also be included.

The pharmaceutical composition of the present invention is administered in a pharmaceutically effective amount. Effective dose levels may be determined depending on types of disease of the patient, severity, activity of drug, sensitivity to drug, administration time, administration route and rate of release, duration of treatment, factors including concurrent medications, and other factors well known in the medical field. The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The dosage of the pharmaceutical composition according to the present invention may vary widely depending on the weight, age, sex, health conditions or diet of a patient, administration time, administration method, excretion rate and severity of the disease, and the appropriate dosage depends on, for example, an amount of drug accumulated in the patient's body and/or specific efficacy of the carrier of the present invention used. Generally, the amount may be calculated on the basis of EC50, which is generally determined to be effective in in *vivo* animal models and *in vitro,* for example, from 0.01 pg to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

Further, the pharmaceutical composition of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

Hereinafter, the present invention will be described in detail with reference to the following examples.

## EXAMPLE

**Example 1. Preparation of artificial chaperones based on porous nanoparticles (ACPNs)**

**1. Preparation of porous artificial chaperone particles**

**(1) Preparation of porous artificial chaperone particles**

**1) Preparation of small pore particles**

960 mL of distilled water (DW) and 810 mL of MeOH were put into a 2 L round bottom flask. 7.88 g of CTAB was added to the flask, followed by rapid addition of 4.52 mL of 1 M NaOH under stirring. After adding a homogeneous mixture while stirring for 10 minutes, 2.6 mL of TMOS was further added. After stirring for 6 hours to mix uniformly, the reaction solution was aged for 24 hours.

Then, the reaction solution was centrifuged at 8000 rpm and 25 °C for 10 minutes to remove the supernatant, centrifuged at 8000 rpm and 25 °C for 10 minutes, and washed five times with ethanol and distilled water alternately.

Thereafter, the resultant product was dried in an oven at 70 °C to harvest 1.5 g of powdery microporous artificial chaperone particles (pore average diameter of 2 nm and particle size of 200 nm).

**2) Pore expansion**

1.5 g of microporous artificial chaperone particle powder was added to 10 ml of ethanol and subjected to ultrasonic dispersion, and 10 ml of water and 10 ml of TMB (trimethyl benzene) were further added, followed by ultrasonic dispersion.

Thereafter, the dispersion was placed in an autoclave and reacted at 160 °C for 48 hours.

The reaction was initiated at 25 °C and performed while raising the temperature at a rate of 10 °C/min, then slowly cooled in an autoclave at a rate of 1 to 10 °C/min.

The cooled reaction solution was centrifuged at 8000 rpm for 10 minutes at 25 °C to remove the supernatant, and centrifuged at 8000 rpm for 10 minutes at 25 °C and washed five times with ethanol and distilled water alternately.

Then, the product was dried in an oven at 70 °C to harvest powdery porous artificial chaperone particles (pore diameter of 10 to 15 nm, and particle size of 200 nm).

**3) Calcination**

The porous artificial chaperone particles prepared in 2) were put in a glass vial, heated at 550 °C for 5 hours, and cooled slowly to room temperature after completing the reaction to prepare particles.

**(2) Preparation of porous artificial chaperone particles**

Porous artificial chaperone particles prepared by the same method as Example 1-1-(1), except that the reaction conditions at the time of pore expansion were changed to 140 °C and 72 hours.

**(3) Preparation of porous artificial chaperone particles (10 L scale)**

Porous artificial chaperone particles were prepared by the same method as Example 1-1-(1), except that a 5 times larger container was used and each material was used in a 5 times capacity.

**(4) Preparation of porous artificial chaperone particles (particle size of 300 nm)**

Porous artificial chaperone particles were prepared by the same method as in Example 1-1-(1), except that 920 ml of distilled water and 850 ml of methanol were used to prepare the small pore particles.

**(5) Preparation of porous artificial chaperone particles (particle size of 500 nm)**

Porous artificial chaperone particles were prepared by the same method as Example 1-1-(1), except that 800 ml of distilled water, 1010 ml of methanol, and 10.6 g of CTAB were used to prepare the small pore particles.

**(6) Preparation of porous artificial chaperone particles (particle size of 1000 nm)**

Porous artificial chaperone particles were prepared by the same method as Example 1-1-(1), except that 620 ml of distilled water, 1380 ml of methanol, and 7.88 g of CTAB were used to prepare the small pore particles.

**(7) Preparation of porous artificial chaperone particles (pore diameter of 4 nm)**

Porous artificial chaperone particles were prepared by the same method as Example 1-1-(1), except that 2.5 mL of TMB was used for pore expansion.

**(8) Preparation of porous artificial chaperone particles (pore diameter of 7 nm)**

Porous artificial chaperone particles were prepared by the same method as Example 1-1-(1), except that 4.5 mL of TMB was used for pore expansion.

**(9) Preparation of porous artificial chaperone particles (pore diameter of 17 nm)**

Porous artificial chaperone particles were prepared by the same method as Example 1-1-(1), except that 11 mL of TMB was used for pore expansion.

**(10) Preparation of porous artificial chaperone particles (pore diameter of 23 nm)**

Porous artificial chaperone particles were prepared by the same method as Example 1-1-(1), except that 12.5 mL of TMB was used for pore expansion.

**(11) Preparation of porous artificial chaperone particles (dual modification)**

**1) Preparation of small pore particles**

Small pore particles were prepared by the same method as Example 1-1- (1) -1).

**2) Pore expansion**

Small pore particles were reacted with TMB, cooled and centrifuged by the same method as Example 1-1-(1)-2) to remove the supernatant. Thereafter, the remaining solution was centrifuged under the same conditions as Example 1-1-(1)-2), washed three times with ethanol and distilled water alternately, and then dried under the same conditions as Example 1-1-(1)-2), thereby harvesting powdery porous artificial chaperone particles (pore diameter 10 to 15 nm, and particle size of 200 nm).

**3) Surface modification**

After dispersing 0.8 g to 1 g of porous artificial chaperone particles having expanded pores in 50 mL of toluene, 5 mL of (3-aminopropyl)triethoxysilane was added thereto, followed by heating under reflux at 120 °C for 12 hours. The procedure is followed by the washing and drying procedures described above, followed by 1 mL of triethylene glycol (PEG3, 2-[2-(2-methoxyethoxy)ethoxy] acetic acid) and 100 mg of EDC (1-ethyl-3-(3-dimethylaminopropyl)carbodiimide) and 200 mg of N-hydroxysuccinimide (NHS) were dispersed in 30 mL of PBS and allowed to react at room temperature for 12 hours under stirring. The product was then washed and dried.
Since the reaction solution of the previous step remained inside of the pores, the inside of the pores was not modified.

**4) Washing inside pores**

800 mg of surface-modified particle powder was dissolved in 40 ml of 2M HCl/ethanol and refluxed under vigorous stirring for 12 hours.
Thereafter, the cooled reaction solution was centrifuged at 8000 rpm for 10 minutes to remove the supernatant, centrifuged at 8000 rpm and 25 °C for 10 minutes, and washed five times with ethanol and distilled water alternately.

Thereafter, the product was dried in an oven at 70 °C, thereby harvesting powdery porous artificial chaperone particles.

**5) Modifying inside pores**

① A propyl group was introduced into the pore by the same method as Example 1-2-(2)-1) described below
② An octyl group was introduced into the pore by the same method as Example 1-2-(2)-2) described below

**2. Surface modification of porous artificial chaperone particles**

**(1) Positively charging**

**1) Particles with particle size of 300 nm**

The porous artificial chaperone particles of Example 1-1-(4) were reacted with (3-aminopropyl)triethoxysilane (APTES) to be positively charged.

Specifically, 100 mg of porous artificial chaperone particles were dispersed in a 10 mL toluene in a 100 mL round bottom flask with a bath sonicator. Then, 1 mL of APTES was added and stirred at 400 rpm and 130 °C for 12 hours.

After the reaction, the product was slowly cooled to room temperature and centrifuged at 8000 rpm for 10 minutes to remove the supernatant, further centrifuged at 8000 rpm and 25 °C for 10 minutes, and then washed five times with ethanol and distilled water alternately.

Thereafter, the product was dried in an oven at 70 °C to harvest powdery porous artificial chaperone particles having an amino group on the surface thereof and inside of the pores.

**2) Particles with particle size of 200 nm**

① The porous artificial chaperone particles of Example 1-1-(1) were positively charged by reacting the particles with (3-aminopropyl)triethoxysilane (APTES), and were modified in the same manner as the method of Example 1-2-(1)-1), except that 0.4 ml of APTES was added and the reaction time was 3 hours.
② The porous artificial chaperone particles of Example 1-1-(9) were positively charged by reacting the particles with (3-aminopropyl)triethoxysilane (APTES), and were modified in the same manner as the method of Example 1-2-(1)-1).
③ The porous artificial chaperone particles of Example 1-1-(10) were positively charged by reacting the particles with (3-aminopropyl)triethoxysilane (APTES), and were modified in the same manner as the method of Example 1-2-(1)-1).

**(2) Introduction of hydrophobic groups**

**1) Propyl group**

The porous artificial chaperone particles of Example 1-1-(1) were reacted with trimethoxy(propyl)silane to introduce propyl groups into the surface of the particles and inside of the pores, and were subjected to modification by the same method as Example 1-2-(1), except that 0.35 ml of trimethoxy(propyl)silane was added instead of APTES, followed by 12 hours of reaction

**2) Octyl group**

The porous artificial chaperone particles of Example 1-1-(1) were reacted with trimethoxy-n-octylsilane to introduce propyl groups on the surface of the particles and inside of the pores, and were subjected to modification by the same method as Example 1-2-(1), except that 0.5 ml of trimethoxy-n-octylsilane was added instead of APTES, followed by 12 hours of reaction.

### (3) Negatively charging

### 1) Carboxyl group

The porous artificial chaperone particles of Example 1-1-(1) were negatively charged by reacting the particles with succinic anhydride. Further, the charged particles were subjected to modification in the same manner as the method of Example 1-2-(1)-1), except that DMSO (dimethyl sulfoxide) was used instead of toluene, 80 mg of succinic anhydride was added instead of APTES to allow reaction at room temperature for 24 hours under stirring, and DMSO was used instead of distilled water.

### 2) Thiol group

The particles were subjected to modification in the same manner as the method of Example 1-2-(1)-1), except that 1.1 mL of MPTES was used instead of APTES.

### 3) Sulfonic acid group

100 mg of the porous silica nanoparticles of Example 1-2-(3)-2) were dispersed in 1 mL of 1 M aqueous sulfuric acid solution and 20 mL of 30% hydrogen peroxide solution, and stirred at room temperature to induce oxidation, thereby oxidizing a thiol group into a sulfonic acid group. Thereafter, the product was washed and dried in the same manner as the method of Example 1-2-(1)-1).

### 3. Identification of particle formation and pore expansion

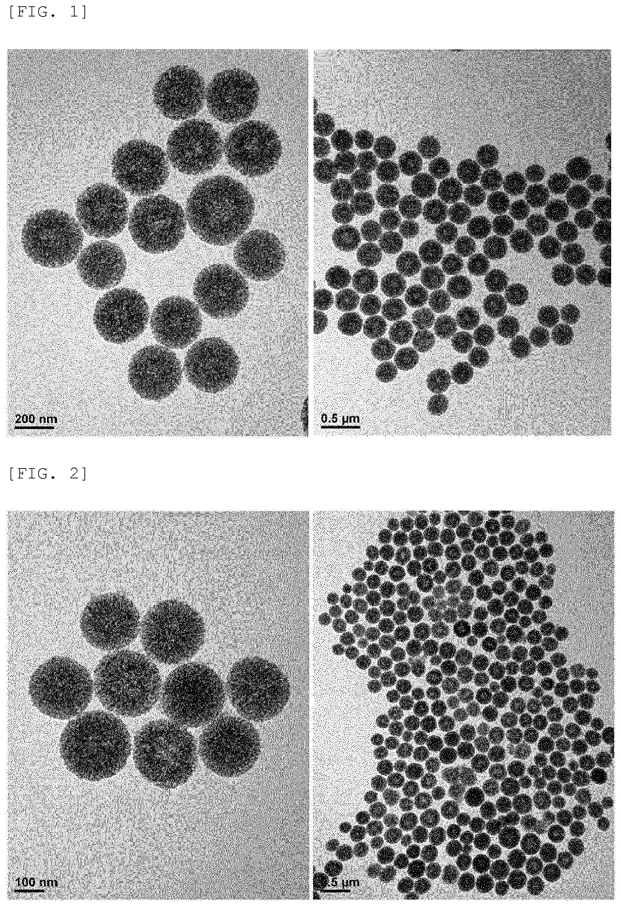

Small pore particles and porous artificial chaperone particles prepared in Experimental Examples 1-1-(1) to (3) were observed under a microscope to determine whether the small pore particles were uniformly formed or the pores were sufficiently expanded to uniformly form the porous artificial chaperone particles (FIGS. 1 to 4).

FIG. 1 is photographs of the porous artificial chaperone particles in Example 1-1-(1), and FIG. 2 is photographs of the porous artificial chaperone particles in Example 1-1-(2), and from these drawings, it can be seen that spherical porous artificial chaperone particles having sufficiently expanded pores were formed evenly.

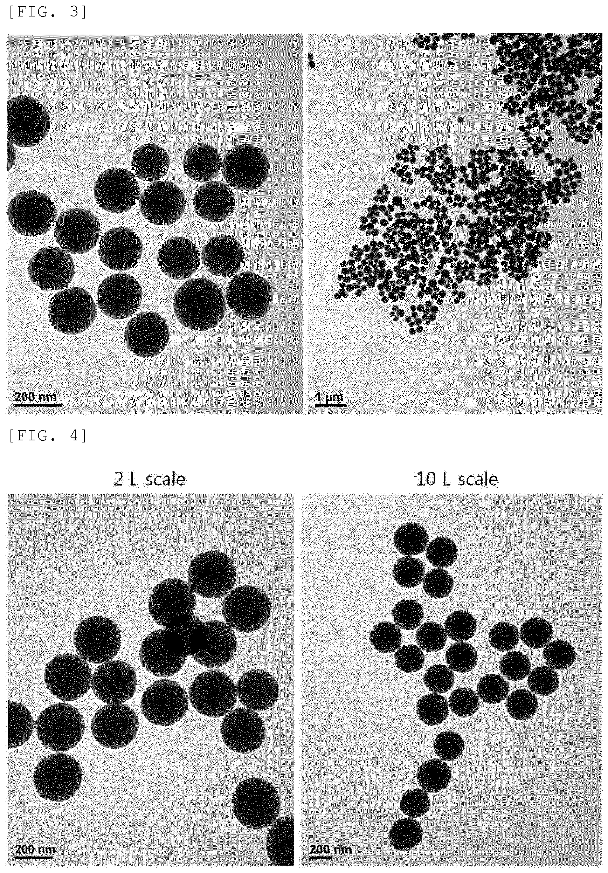

FIG. 3 is photographs of the small pore particles in Example 1-1-(1), and FIG. 4 is comparative photographs of the small pore particles in Examples 1-1-(1) and 1-1-(3), and from these drawings, it can be seen that spherical small pore particles were formed evenly.

### 4. Calculation of BET surface area and pore volume

Surface areas and pore volumes of the small pore particles in Example 1-1-(1) and the porous artificial chaperone particles of Examples 1-1-(1), (7), (8) and (10) were calculated. The surface areas were calculated by Brunauer-Emmett-Teller (BET) method, and the pore size distributions were calculated by Barrett-Joyner-Halenda (BJH) method.

Micrographs of the particles are shown in FIG. 5, and the calculation results are shown in Table 1 below.

[TABLE 1]

| Section | Pore diameter (nm) | BET surface area ($m^2$/g) | Pore volume (mL/g) |
|---|---|---|---|
| Small pore particle in Example 1-1-(1) | 2.1 | 1337 | 0.69 |
| Example 1-1-(7) | 4.3 | 630 | 0.72 |
| Example 1-1-(8 ) | 6.9 | 521 | 0.79 |
| Example 1-1-(1) | 10.4 | 486 | 0.82 |
| Example 1-1-(10) | 23 | 395 | 0.97 |

### 5. Identification of biodegradability

In order to identify biodegradability of the porous artificial chaperone particles in Example 1-1-(1), biodegradability at 37 °C in SBF (pH 7.4) was observed under a microscope at 0 hours, 120 hours and 360 hours, and results thereof are shown in FIG. 6.

Referring to FIG. 6, it can be seen that the porous artificial chaperone particles are biodegraded and almost degraded after 360 hours.

**6.** Measurement of absorbance ratio

Absorbance ratio over time was measured according to Equation 1 below.

$$[\text{Equation 1}]$$

$$A_t/A_0$$

(wherein $A_0$ is absorbance of the porous artificial chaperone particles measured by putting 5 ml of suspension containing 1 mg/ml of the porous artificial chaperone particles into a cylindrical permeable membrane having pores with a pore diameter of 50 kDa,

15 ml of the same solvent as the suspension comes into contact with an outside of the permeable membrane, and the inside/outside of the permeable membrane are horizontally stirred at 60 rpm and 37 °C, and

$A_t$ indicates absorbance of the porous artificial chaperone particles measured after lapse of "t" hours since $A_0$ was measured).

Specifically, 5 mg of porous artificial chaperone particle powder was dissolved in 5 ml of SBF (pH 7.4). Thereafter, 5 ml of porous artificial chaperone particle solution was placed in a permeable membrane having pores with a pore diameter of 50 kDa shown in FIG. 7. 15 ml of SBF was added to the outer membrane, and the SBF on the outer membrane was replaced every 12 hours. Degradation of the porous artificial chaperone particles was performed at 37 °C under horizontal stirring at 60 rpm.

Then, the absorbance was measured by UV-vis spectroscopy and analyzed at $\lambda$ = 640 nm.

**(1) Measurement of absorbance ratio**

Absorbance ratio of the porous artificial chaperone particles in Example 1-1-(1) was measured according to the above method, and results thereof are shown in FIG. 8.

Referring to FIG. 8, it can be seen that t, at which the absorbance ratio becomes 1/2, is about 58 hours to demonstrate very slow degradation.

**(2) Particle size**

Absorbances of the porous artificial chaperone particles in Examples 1-1-(1), (5) and (6) were measured according to Equation 1 above, and results thereof are shown in FIG. 9 (SBF used as the suspension and the solvent).

Referring to FIG. 9, it can be seen that t is decreased as the particle size is increased.

**(3) Average pore diameter**

Absorbances of the porous artificial chaperone particles in Examples 1-1-(1) and (9) and the microporous artificial chaperone particles in Example 1-1-(1) as a control were measured according to Equation 1 above, and results thereof are shown in FIG. 10 (SBF used as the suspension and the solvent).

Referring to FIG 10, it can be seen that the porous artificial chaperone particles of the inventive example have a significantly larger t than the control.

**(4) pH**

Absorbance of the porous artificial chaperone particles in Example 1-1-(4) for each pH was measured. The absorbance was measured in SBF and in Tris at pH 2, 5, and 7.4, and results thereof are shown in FIG. 11.

Referring to FIG 11, it could be seen that, although there is a difference in t in relation to pH, t at which all absorbance ratio becomes 1/2 was 24 or more.

**(5) Charging**

Absorbance of the porous artificial chaperone particles in Example 1-2-(1)-1 was measured, and results thereof are shown in FIG. 12 (Tris (pH 7.4) used as the suspension and the solvent).

Referring to FIG 12, it could be seen that t at which the absorbance ratio of the positively charged particles becomes 1/2 was 24 or more.

## Example 2. Stapling of polypeptides

### 1. Assessment of designs and characteristics of porous artificial chaperone particles

As the porous artificial chaperone particles, the porous artificial chaperone particles of Example 1-1-(1) without surface modification, the particles of Example 1-2-(2)-1 having a propyl group introduced therein, and the particles of Example 1-2-(2)-2 having an octyl group introduced therein were used.

Each ACPN (10 mg) was suspended in 100 $\mu$l of Nile Red solution (1 mg/ml methanol solution) and stirred for 1 hour. These complexes were collected by centrifugation and washed alternately with methanol and water several times to remove the Nile Red adsorbed to the particle surface. Each ACPNs-Nile Red complex solution was made with a constant Nile Red content using UV/vis absorption measurements. A fluorescence emitting spectrum of Nile Red encapsulated in the pores inside the particles was measured ($\lambda$ex = 510 nm) and the relative emission intensity was compared for each composite solution.

It was confirmed by TEM image and dynamic light scattering (DLS) that there was no significant change in the particle size and size distribution before and after loading p53 peptide (Formula 1) to the particles (FIG. 14).

[Formula 1]          FAM-GGQSQQTF*KNLWRLL**KQN-NH$_2$

(*K : d-Lysine conjugated pentynoic acid, **K : 2-amino-5-azido-pntanoic acid)

### 2. Assessment of stapling extent of polypeptides according to difference in surface modification

CD spectra were measured using a J-810 spectropolarimeter (JASCO, Japan). Spectra were recorded at wavelengths in a range of 250 to 190 nm using a 1 cm path length cuvette. The test was repeated five times and averaged. The ACPN suspension concentration was 0.2 mg/mL (p53 concentration: 20 $\mu$M). All sample suspensions were stabilized for at least 2 days at room temperature before CD measurement. Spectra were scanned at 10 °C interval in a temperature range of 25 to 75 °C in order to implement CD experiments relative to temperature. $\alpha$-helicity of the peptide was calculated using CONTIN-LL algorithm.

When substituted with an octyl group, the highest alpha helical level stabilization was demonstrated (FIG. 15) .

### 3. Cell experiment

Human liver cancer cell-line HepG2 and human cervical cancer cell-line HeLa were grown in a humidified atmosphere of 37 °C, 5% CO$_2$ in DMEM containing 10% FBS and 1% penicillin/streptomycin. After incubating each cell for 24 hours in a 4-well glass plate, ACPN with p53 (0.02 mg/ml) and free clicked p53 (2 $\mu$M) were added to each well containing a serum-free medium followed by incubation for 12 hours. The cells were carefully rinsed with 1 $\times$ PBS and the medium was replaced with a serum containing medium. After staining nuclei with Hoechst 33342, brightfield and fluorescence images were obtained using a DeltaVision Elite Microscopy System (GE Healthcare, USA). The nuclei were treated with TAMRA-conjugated ACPNP-C8 carried with clicked p53 (0.25 mg/ml) and, in order to confirm co-location between fluorescent signals over time, Mander's overlap coefficients were calculated by JACoP plug-in in the Image J program. HepG2 and HeLa cells (1 $\times$ 10$^4$ cells/well) were incubated for 24 hours in 96-well plates, followed by addition at various concentrations of ACPN and ACPN complexes. After incubation, the cells were carefully rinsed with 1 $\times$ PBS. Cell viability was estimated using CCK-8 assay. EC50 values were determined by Logistic curve fitting method of Origin (OriginLab, USA).

Caspase-3 activity assay was performed to identify effects of inducing apoptosis by ACPNP-C8@clicked p53. HepG2 cells were treated with ACPNP-C8, ACPNP-C8@clicked p53 (12.5 $\mu$M, 25 $\mu$M) and PBS, respectively, for 4 hours, followed by incubation with a fresh medium for 20 hours. The cells were washed, treated with a lysis buffer (Promega, USA) and centrifuged at 4 °C for 10 minutes. Collected lysate was incubated with caspase-3 substrate (Ac-DEVD-pNA, 100 $\mu$M) in a humidified incubator at 37 °C for 2 hours. Fluorescence intensities were then measured ($\lambda$ex = 400nm, $\lambda$em = 505nm).

Referring to FIG. 16, it can be seen that ACPNP-C8@clicked p53 has significantly superior activity.

### 4. In *vivo* experiment

Five week old Balb/c male nude mice were injected subcutaneously with a suspension of HepG2 cells (6 $\times$

$10^6$ cells) and the tumor bearing mice were bred until the tumor volume of HepG2 reaches up to 50 $mm^3$. In order to investigate tumor growth inhibition efficiency, ACPN-C8@clicked p53, ACPN-C8 and clicked p53 (50 mg ACPN-C8/kg, 5 $\mu$mole clicked p53/kg) in 100 $\mu$L of 1 $\times$PBS solution, respectively, were directly injected into the tumor (IT injection). An equal volume of 1 $\times$PBS solution was also injected directly into the tumor as a control. Intratumoral injections were performed at 5 day interval and changes in tumor volume and body weight were monitored for 12 days. Tumor volume was calculated relative to the initial tumor volume. For imaging experiments, TAMRA-labeled ACPN-C8 was treated in the same manner as above instead of ACPN-C8. Fluorescence images of mice were acquired over time for 3 days using an *in vivo* imaging system FoBI (Neoscience, Korea).

Referring to FIG. 17, it can be seen that ACPNP-C8@clilcked p53 has significantly superior activity.

## 5. Polypeptide release

Clicked p53 peptide release experiments from ACPN-C8 were conducted in PBS buffer and PBS buffer containing 10% FBS, respectively. ACPN-C8 carried with clicked p53 (1 mg/ml) was dispersed in each of PBS buffer solution and 10% FBS-containing PBS buffer solution, and then incubated under stirring at 37 °C (N=3). At each time, the supernatant was collected by centrifugation, and fluorescent intensities ($\lambda$ex = 480 nm, $\lambda$em = 520 nm) of FAM-labeled clicked p53 from the supernatant were measured so as to calculate a concentration of released peptide.

Referring to FIG. 18, it can be seen that a release rate of polypeptide is significantly slow in the buffer solution containing no FBS.

## Claims

1.  A composition for delivery of polypeptide, comprising: porous artificial chaperone particles having an average pore diameter of 1 to 100 nm.

2.  The composition according to claim 1, wherein the particles are used to staple the polypeptide supported inside of pores thereof.

3.  The composition according to claim 1, wherein an average diameter of the porous artificial chaperone particles ranges from 150 to 1000 nm.

4.  The composition according to claim 1, wherein a BET surface area of the porous artificial chaperone particles ranges from 200 to 700 $m^2$/g, and a volume per gram of the pores ranges from 0.7 to 2.2 ml.

5.  The composition according to claim 1, wherein the porous artificial chaperone particles are prepared by: reacting silica particles having pores with a pore diameter of less than 5 nm with a swelling agent at 120 to 180 °C for 24 to 96 hours to expand the pores having a pore diameter of less than 5 nm; and calcining the silica particles having expanded pores at a temperature of 400 °C or higher for at least 3 hours.

6.  The composition according to claim 1, wherein the porous artificial chaperone particles are **characterized in that** t, at which an absorbance ratio in the following Equation 1 becomes 1/2, is 24 or more:

$$[Equation\ 1]$$

$$A_t/A_0$$

   (wherein $A_0$ is absorbance of the porous artificial chaperone particles measured by putting 5 ml of suspension containing 1 mg/ml of porous artificial chaperone particles into a cylindrical permeable membrane having pores with a pore diameter of 50 kDa,
   15 ml of the same solvent as the suspension comes into contact with an outside of the permeable membrane, and the inside/outside of the permeable membrane are horizontally stirred at 60 rpm and at 37 °C,
   pH of the suspension is 7.4, and
   indicates absorbance of the porous artificial chaperone particle measured after lapse of "t" hours since $A_0$ was measured) .

7.  The composition according to claim 1, wherein the porous artificial chaperone particles have a hydrophilic substituent on an outer surface thereof and a hydrophobic substituent on an inside of the pores.

8. The composition according to claim 1, wherein the polypeptide is an alpha helical polypeptide or an enzyme polypeptide.

9. The composition according to claim 8, wherein the alpha helical polypeptide is at least one selected from the group consisting of Bcl-2 family protein inhibitor, MDM2/MDMX inhibitor, HIV envelope protein gp41 inhibitor, epidermal growth factor receptor inhibitor, Kirsten rat sarcoma viral oncogene homolog (KRAS) inhibitor, Rab (Ras-related in brain) inhibitor, and myoA (malaria invasion motor myosin) tail interacting protein (MTIP) inhibitor.

10. The composition according to claim 8, wherein the enzyme polypeptide is at least one selected from the group consisting of carbonic anhydrase, lysozyme, insulin, BSA, lipase, amylase, chymotrypsin, citrate synthase, formate dehydrogenase, GFP and mitochondrial malate dehydrogenase (mMDH).

11. The composition according to claim 1, wherein the polypeptide is p53.

12. A composition for delivery of polypeptide, comprising:

porous artificial chaperone particles having an average pore diameter of 1 to 100 nm; and
a polypeptide supported and stapled inside of pores of the particle.

13. The composition according to claim 12, wherein the porous artificial chaperone particles have an average diameter of 150 to 1000 nm.

14. The composition according to claim 12, wherein a BET surface area of the porous artificial chaperone particles ranges from 200 to 700 $m^2/g$ and a volume per g of pores ranges from 0.7 to 2.2 ml

15. The composition according to claim 12, wherein the porous artificial chaperone particles are prepared by: reacting silica particles which have pores having a pore diameter of less than 5 nm with a swelling agent at 120 to 180 °C for 24 to 96 hours to expand the pores having a pore diameter of less than 5 nm; and calcining the silica particles having expanded pores at a temperature of 400 °C or higher for at least 3 hours.

16. The composition according to claim 12, wherein the porous artificial chaperone particles are **characterized in that** t, at which an absorbance ratio in the following Equation 1 becomes 1/2, is 24 or more:

$$[Equation\ 1]$$

$$A_t/A_0$$

(wherein $A_0$ is absorbance of the porous artificial chaperone particles measured by putting 5 ml of suspension containing 1 mg/ml of porous artificial chaperone particles into a cylindrical permeable membrane having pores with a pore diameter of 50 kDa,
15 ml of the same solvent as the suspension comes into contact with an outside of the permeable membrane, and the inside/outside of the permeable membrane are horizontally stirred at 60 rpm and at 37 °C,
pH of the suspension is 7.4, and
indicates absorbance of the porous artificial chaperone particle measured after lapse of "t" hours since $A_0$ was measured).

17. The composition according to claim 12, wherein the porous artificial chaperone particles have a hydrophilic substituent on an outer surface thereof and a hydrophobic substituent on an inside of the pores.

18. The composition according to claim 12, wherein the polypeptide has an alpha-helix structure on the inside of the pores.

19. The composition according to claim 12, wherein the polypeptide is an alpha helical polypeptide or an enzyme polypeptide.

20. The composition according to claim 19, wherein the alpha helical polypeptide is at least one selected from the group consisting of Bcl-2 family protein inhibitor, MDM2/MDMX inhibitor, HIV envelope protein gp41 inhibitor, epidermal growth factor receptor inhibitor, Kirsten rat sarcoma viral oncogene homolog (KRAS) inhibitor, Rab (Ras-related in brain) inhibitor, and myoA (malaria invasion motor myosin) tail interacting protein (MTIP) inhibitor.

21. The composition according to claim 19, wherein the enzyme polypeptide is at least one selected from the group consisting of carbonic anhydrase, lysozyme, insulin, BSA, lipase, amylase, chymotrypsin, citrate synthase, formate dehydrogenase, GFP and mitochondrial malate dehydrogenase (mMDH).

22. The composition according to claim 12, wherein the polypeptide is p53.

23. A method for stapling polypeptide, comprising: mixing porous artificial chaperone particles which have an average pore diameter of 1 to 100 nm and polypeptide so as to carry the polypeptide inside the pores; and stapling the same.

[FIG. 1]

[FIG. 2]

[FIG. 3]

[FIG. 4]

2 L scale          10 L scale

[FIG. 5]

[FIG. 6]

[FIG. 7]

[FIG. 8]

[FIG. 9]

| Sample | $t_{50\%}$ |
|---|---|
| DDV(200)$_{10}$ | 57.4 h |
| DDV(500)$_{10}$ | 37.5 h |
| DDV(1000)$_{10}$ | 30.0 h |

[FIG. 10]

| Sample | $t_{50\%}$ |
|--------|-----------|
| MSN(200)$_2$ | 17.9 h |
| DDV(200)$_{10}$ | 57.4 h |
| DDV(200)$_{17}$ | 53.6 h |

[FIG. 11]

[FIG. 12]

[FIG. 13]

[FIG. 13]

**a**

R: OH, CH₂CH₂CH₃ (**C3**), CH₂(CH₂)₆CH₃ (**C8**)

17 nm

**b**

645 nm
640 nm
633 nm

——— ACPN-OH
– – – ACPN-C3
· · · · · ACPN-C8

Rel. Fluo. Int. (a.u.)

Wavelength (nm)

**c**

ACPN-C8          ACPN-C8@p53

**d**

■ ACPN-C8
▨ ACPN-C8@p53

Intensity (%)

Diameter (nm)

[FIG. 14]

EP 3 838 924 A2

[FIG. 12]

**DDV(300)$_{17}$–NH$_2$**

t$_{50\%}$ = About 2.5 days

[FIG. 13]

[FIG. 14]

a

R: OH, CH₂CH₂CH₃ (C3), CH₂(CH₂)₆CH₃ (C8)

b

c

ACPN-C8          ACPN-C8@p53

d

[FIG. 15]

| α-helicity % | p53 | clicked p53 |
|---|---|---|
| **ACPN-OH** | 46.6 | 52.4 |
| **ACPN-C3** | 60.7 | 68.6 |
| **ACPN-C8** | 69.1 | 84.9 |

[FIG. 16a]

[FIG. 16b]

[FIG. 16c]

[FIG. 17]

[FIG. 17a]

**a**

[FIG. 17c]

**c**

[FIG. 17]

[FIG. 18]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REMINGTON.** Pharmaceutical Science. Mack Publishing Company